(19) Europäisches Patentamt
European Patent Office
Office européen des brevets

(11) Publication number: **0 475 230 A1**

(12) **EUROPEAN PATENT APPLICATION**

(21) Application number: **91114726.2**

(22) Date of filing: **02.09.91**

(51) Int. Cl.[5]: **C07K 5/12**, A61K 39/395, A61K 39/44, A61K 39/385

(30) Priority: **11.09.90 US 580835**

(43) Date of publication of application: **18.03.92 Bulletin 92/12**

(84) Designated Contracting States: **CH DE FR GB IT LI SE**

(71) Applicant: **BRUNSWICK CORPORATION**
**One Brunswick Plaza**
**Skokie Illinois 60077(US)**

(72) Inventor: **Wrasidlo, Wolfgang A.**
**307 Prospect Street**
**La Jolla, California 92037(US)**

(74) Representative: **UEXKÜLL & STOLBERG**
**Patentanwälte**
**Beselerstrasse 4**
**W-2000 Hamburg 52(DE)**

(54) **Methods of conjugating actinomycin D.**

(57) The invention provides for actinomycin D derivatives and methods of producing actinomycin D derivatives. Such derivatives include isocyanate, thioisocyanate, carboxylic acid, bromide, aldehyde and sulfonic acid derivatives. The invention also provides for actinomycin D derivatives conjugated to target cell binding proteins. Methods are provided to make such conjugates through attachment of the derivatized moiety to a reactive group on a target cell binding protein or on a spacer. The conjugates can be administered to an animal and produce localized cytotoxic effects on target cells.

FIGURE 1

A.

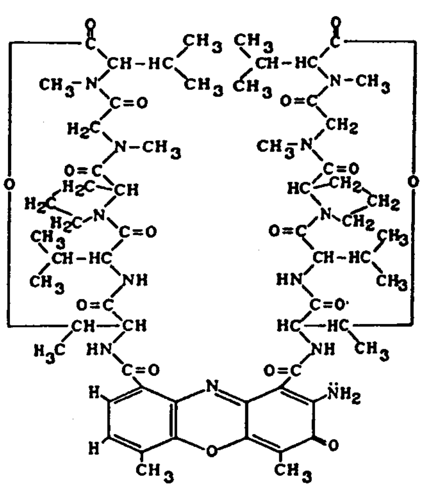

B.

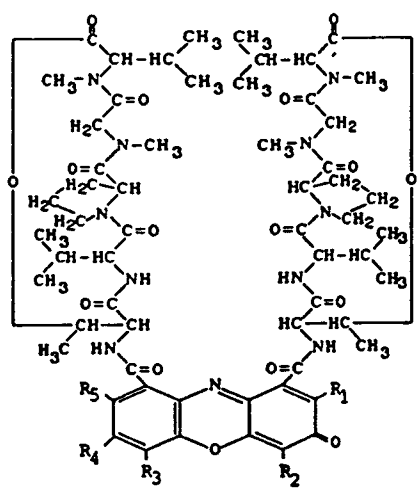

EP 0 475 230 A1

## BACKGROUND OF THE INVENTION

This invention relates to conjugates of antibodies and therapeutic agents and, more specifically, to therapeutic preparations of actinomycin D linked to an antibody for delivery of actinomycin D to the site of action as dictated by the antibody specificity.

It has long been recognized as highly desirable to specifically target therapeutic agents to invading organisms or diseased cells. Specific targeting permits lover doses of the therapeutic agent to be given and reduces the observed side effects from non-specific action of the agent. Various target cell binding agents, such as liposomes, proteins and antibodies, have been used in conjunction with pharmaceutical or cytotoxic agents, such as microbial toxins, protein synthesis inhibitors (i.e. diphtheria toxin) and radioactive compounds as specific targeting molecules.

Antibodies, particularly monoclonal antibodies, which recognize specifically selected antigens are especially suited for delivery of therapeutic agents. Monoclonal antibodies are advantageous since they have the ability to recognize a single molecular site or epitope on a cell. Studies have identified monoclonal antibodies specifically directed toward tumor-associated antigens and other antigens on cancer cells, T-cells and B-cells. These monoclonal antibodies can be used to deliver drugs directly and specifically to the target cells. One of the potential means of attacking such target cells is to use the monoclonal antibodies with an attached drug, such as an antibiotic. Delivery of antibody-therapeutic agent to specific cells, tissues, organs, or any other site in vivo can be accomplished using whole antibodies or fragments of antibodies. Fragments, such as Fab, can be used in place of whole antibodies if they retain the ability to recognize selected antigens.

Conjugates of antibody-therapeutic agent can be made by chemically coupling the two through covalent bonds. One disadvantage of covalent attachment to the backbone of an antibody molecule is that if the chemical modification is in the antigen binding region, the recognition of the antibody can be changed. This adverse effect on the functional properties of the antibody has been a problem with the random linkage of drugs to antibodies. The critical features of the resulting conjugate are that it maintain its biological activity, (both the antibody and therapeutic agent) and that it be stable for use in vivo. The attachment of the therapeutic agent to the target cell binding protein must be stable in all conditions of administration to a patient and under all conditions present in the microenvironment at the site of action. Further, for administration of an effective amount in a human or animal, the conjugate must remain immunospecific for an antigenic determinant on specific cells or tissues.

Actinomycin D is an antibiotic which inhibits RNA transcription, the process by which genetic information in one strand of DNA is copied into a complementary set of bases called messenger RNA (mRNA). Actinomycin D intercalates, or inserts itself, into the double-helical DNA between G≡C base pairs causing a deformed DNA template, thereby preventing efficient RNA transcription. In essence, actinomycin D jams the DNA zipper.

Further, actinomycin D is especially attractive due to its effectiveness at low concentrations. In low concentrations, actinomycin D inhibits transcription without appreciably affecting DNA replication or protein synthesis. It has been extensively used as a highly specific inhibitor of the formation of new RNA in both procaryotic and eucaryotic cells which eventually results in the death of the cells. Additionally, its inhibition of the growth of rapidly dividing cells makes it an effective non-selective therapeutic agent in the treatment of some cancers.

Actinomycin D has never been attached with a carrier through any type of linkage. The chemistry for attaching actinomycin D to selective targeting agents has been burdened with unwanted side products and all attempts have been unsuccessful.

There thus exists a need for target cell binding agents conjugated to actinomycin D which can be used for therapeutic or diagnostic purposes. Preferably, such a conjugate should be stable in vivo. The present invention satisfies these needs and provides related advantages as well.

## SUMMARY OF THE INVENTION

The invention provides actinomycin D derivatives and methods of producing actinomycin D derivatives, which are useful for conjugating actinomycin D to other materials. Such derivatives include isocyanate, thioisocyanate, carboxylic acid, bromine, aldehyde and sulfonic acid derivatives. The invention also provides for actinomycin D derivatives conjugated to target cell binding proteins. Methods are provided for synthesizing such conjugates through attachment of the derivative to a reactive group on a target cell binding protein or a spacer. The conjugates can be administered to an animal and produce localized cytotoxic effects on target cells.

## BRIEF DESCRIPTION OF THE FIGURES

Figure 1a shows the structure of actinomycin D. Figure 1b shows locations of the moieties, represented as $R_1$ through $R_5$, on the aromatic component of actinomycin D which can be modified.

Figure 2 shows the in vivo binding and distribution of a $^{125}I$ labeled actinomycin D conjugate. The indicated tissues are: BL, blood; TA, tail; TU, tumor; HE, heart; SK, skin; MU, muscle; BO, bone; LU, lung; LI, liver; SP, spleen; ST, stomach; TH, thyroid; KI, kidney; IN, intestine.

## DETAILED DESCRIPTION OF THE INVENTION

This invention is directed to simple and efficient methods for producing chemical derivatives of actinomycin D and methods for coupling such derivatives to target cell binding proteins to produce actinomycin D conjugates. Until now, such actinomycin D conjugates did not exist because the chemistry for producing actinomycin D derivatives was burdened with unwanted side products. An important advantage of producing chemical derivatives of actinomycin D and actinomycin D conjugates is that the actinomycin D antibiotic is a very potent inhibitor of RNA synthesis in virtually all types of cells. Coupling the actinomycin D derivative to a target cell binding protein allows the specific killing of any cell type to which a target cell binding protein is available. Thus, the actinomycin D conjugates produced from the chemical derivatives of actinomycin D are applicable in specifically suppressing tumor growth in patients and in suppressing contaminating cell growth in culture.

In one embodiment, the method for producing chemical derivatives of actinomycin D involves the formation of an isocyanate derivative of actinomycin D. Actinomycin D has a single primary amine located on the three-ring aromatic component of the molecule. Reacting oxalyl chloride with actinomycin D converts the primary amine into an isocyanate moiety. The isocyanate moiety is reactive with chemical groups on the target cell binding protein such as the hydroxyl moieties of serine and the primary amine moieties of lysine. Coupling of the isocyanate moiety to hydroxyl or primary amine groups on target cell binding proteins produces an actinomycin D conjugate useful for selectively suppressing target cell growth.

Alternatively, the isocyanate derivative of actinomycin D can be coupled to a target cell binding protein through a spacer. The spacer can be attached at one end to the isocyanate moiety through a hydroxyl or primary amine reactive group. The other end of the spacer will have a chemical moiety, such as a carboxylic acid, capable of being coupled to a target cell binding protein.

In other embodiments of the invention, methods for producing a variety of other actinomycin D derivatives are provided. These methods involve the modification of the primary amine of actinomycin D to produce other isocyanate derivatives as well as thioisocyanate and carboxylic acid derivatives. Methods involving the modification of the aromatic methyl group substituents to produce methylene bromide and aldehyde derivatives of actinomycin D are also provided. Additionally, sulfonic acid derivatives of actinomycin D can be produced by replacing the aromatic hydrogen atoms. Each of the above derivatives can be coupled to a target cell binding protein through the derivatized moiety to produce actinomycin D conjugates for use in specifically suppressing target cell growth.

As used herein, the term "target cell binding protein" refers to a protein which exhibits selective binding to a target cell. The protein can be, for example, a monoclonal antibody, polyclonal serum, protein hormone, growth factor and cell surface binding protein such as a cell adhesion protein. The term is also meant to include functional fragments of target cell binding proteins. "Functional fragments," as used herein, refers to fragments which exhibit the selective binding properties to a target cell of the intact molecule. Selective binding indicates binding specificity and affinity of the target cell binding protein for the target cell. The molecule being bound on the target cell is typically a cell surface molecule, such as a protein antigen, although carbohydrate, lipid, inorganic molecules such as phosphate and sulphate, polypeptide and peptide molecules can also be bound as well so long as the target cell binding protein exhibits selective binding for these molecules. The target cell can be any cell or population of cells which contains a cell surface molecule capable of being bound by the target cell binding protein. Cells in culture are included as well as cells within an organism.

As used herein, the term "actinomycin D derivative" refers to a modified form of actinomycin D. Actinomycin D is the compound shown in Figure 1a. There are five moieties on actinomycin D which can be modified. The moieties are located on the three-ring aromatic component of actinomycin D and are replaced in Figure 1b as groups $R_1$ through $R_5$. Reference to these moieties in chemical equations will be diagrammed as the moiety attached to the letters "AMD", where AMD represents the rest of the actinomycin D molecule. For example, $R_1$, which is the primary amine on the aromatic component of actinomycin D, will be represented as AMD-$NH_2$. Modifications of such moieties to produce an actinomycin

D derivative can include, for example, addition of chemical groups or substitution. Actinomycin D derivatives include, for example, isocyanate derivatives, carboxylic acid derivatives, methylene bromide derivatives, thioisocyanate derivatives, aldehyde derivatives, and sulfonic acid derivatives. Such derivatives will also be represented in the same manner as that described above.

As used herein, the term "actinomycin D conjugate" or "conjugate" refers to an actinomycin D derivative coupled to a target cell binding protein. The term "coupled," as used herein, refers to the joining of two molecules together, such as through a covalent bond between an actinomycin D derivative and a target cell binding protein. The two molecules can also be an actinomycin D derivative and a spacer. Therefore, the coupling of an actinomycin D derivative can be through direct coupling to the target cell binding protein or coupling through a spacer molecule.

As used herein, the term "reactive group" refers to a chemical moiety which exhibits bond formation activity. Coupling a molecule through a reactive group results in bond formation between the molecule and the reactive group. Reactive groups can be, for example, primary amines, hydroxyls, carboxylic acids, isocyanates, aldehydes, sulfonic acids, and methylene bromides. Bond formation includes prior activation of the chemical moiety to achieve bond formation.

The term "activation," as used herein, refers to a chemical reaction which results in modification of the chemical reactivity of a chemical moiety. The modification typically does not change greatly the chemical specificity of the moiety, but instead, allows the moiety to be reacted faster, more easily and often with the production of fewer side products. Activation of carboxylic acid moiety with, for example, carbodiimide, N-hydroxysuccinimide, N-ethoxycarbonyl-2-ethoxy-1,2-dihydroquinoline (EEDQ) or isobutyl chloroformate, allows rapid and efficient coupling of the activated carboxylic acid moiety to a primary amine to form an amide bond. Other activating reagents include, for example, isocyanates, thioisocyanates, azides and diazohalides. The preferred activating reagent will depend on the particular chemical moiety involved and on the desired bond to be produced. Such reactions are known to one skilled in the art.

As used herein, the term "spacer" refers to a molecule which forms a bridge-like structure between an actinomycin D derivative and a target cell binding protein. The spacer can be used, for example, as an alternative means for coupling an actinomycin D derivative to a target cell binding protein other than directly through reactive groups on the two molecules. Such spacers can consist of one or more atoms but are preferably less than about twenty atoms in length. Spacers can be bifunctional, or more preferably, heterobifunctional; both types of spacers contain reactive groups on each end of the molecule useful in coupling actinomycin D derivatives to an antibody. Bifunctional spacers have the same reactive group on each end. Heterobifunctional spacers, on the other hand, have different reactive groups at each end. Heterobifunctionality is beneficial because it reduces crosslinking and the production of unwanted side products in the coupling reaction. Heterobifunctional spacers include, for example, hydrocarbons of less than about twenty carbon atoms in length, preferably about ten, with one of a variety of chemical moieties at one end and a different chemical moiety at the other end. Preferably, a carboxylic acid group is included as one of the chemical moieties. The other end can contain one of a variety of chemical moieties which will depend on the chemistry necessary for coupling to an actinomycin D derivative.

Peptide spacers are also included which have, for example, the structure: R-Peptide-$R_1$-COOH. R corresponds to the actinomycin D reactive end and includes such chemical moieties as oximes ($H_2N$-O), phenylhydrazines ($H_2N$-NH-Ph), hydrazides ($H_2N$-NH-CO), and hydrazine-sulfonyl ($H_2N$-NH-$SO_2$-Ph). Other chemical moieties are also included such as hydroxyl, primary amine, aromatic oxygens and brominated methylene groups. $R_1$ corresponds to the target cell binding protein reactive end and can include, for example, structures such as maleic acid, succinic acid, citraconic acid, diglyconic acid, and dimethylmaleic acid. The peptide can be of any length and sequence but is preferably about three to four amino acids in length. The sequence is preferably Ala-Ala-Ala, Ala-Leu-Ala-Leu, Leu-Ala-Leu-Ala or Ser-Ser-Ser.

The invention provides an isocyanate derivative of actinomycin D as well as a method for producing such isocyanate derivatives of actinomycin D. Actinomycin D contains a single primary amine which can be converted to an isocyanate moiety by reaction with a compound such as oxalyl chloride. The reaction with oxalyl chloride is as follows:

$$\text{AMD-NH}_2 + \text{ClCOCOCl} \xrightarrow{-\text{HCl}} \text{AMD-NHCOCOCl} \longrightarrow$$

$$\text{AMD-NCO} + \text{CO} + \text{HCl}$$

where $AMD-NH_2$ represents the primary amine of actinomycin D.

Compounds similar to oxalyl chloride can also be used to produce other isocyanate derivatives of actinomycin D as well as carboxylic acid and thioisocyanate derivatives. Such compounds include, for example, acid chlorides of the structure Cl-CO-R-X where R is a hydrocarbon and X can be an isocyanate, carboxylic acid or thioisocyanate. Reaction of this acid chloride with the primary amine of actinomycin D produces an amide bond between the primary amine and the carbonyl group of the acid chloride. The resultant derivative has the structure AMD-NH-CO-R-X. Other chemical moieties can be used as well, such as an aldehyde group, in place of the acid chloride moiety of the above structure. Therefore, the invention provides for a variety of isocyanate, carboxylic acid and thioisocyanate derivatives as well as methods of producing such derivatives.

The invention also provides for a carboxylic acid derivative of actinomycin D with a structure different than that described above and a method of producing such carboxylic acid derivatives as well. Reaction of actinomycin D with brominated carboxylic acids, such as bromoacetic acid, releases hydrogen bromide and forms a bond between the primary amine located on actinomycin D and the methylene group of bromoacetic acid. The reaction is depicted as follows and results in secondary amine formation:

$AMD-NH_2 + BrCH_2CO_2H$ ----> $AMD-NH-CH_2-CO_2H + HBr$.

Halogen atoms other than bromine can also be used in the carboxylic acid reactant. Such halogen atoms can be, for example, chloro or iodo and are known to those skilled in the art.

The invention further provides for a methylene bromide derivative of actinomycin D as well as a method of producing such a derivative. Reaction of bromine or bromotrichloromethane, for example, with actinomycin D in the presence of ultraviolet light brominates the methyl group substituents of the aromatic rings. Alternatively, bromination, such as that shown below, will also result in a methylene bromide derivative of actinomycin D.

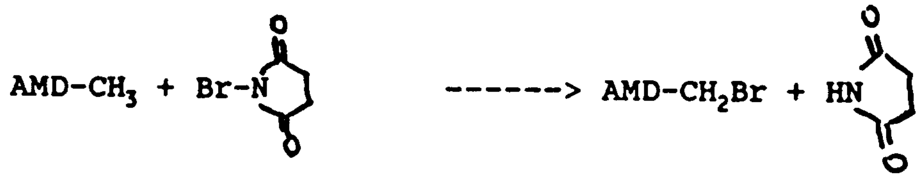

where $AMD-CH_3$ represents either of the two aromatic methyl group substituents on actinomycin D.

The invention further provides for aldehyde derivatives of actinomycin D and methods for producing such derivatives. The aromatic methyl group substituents of actinomycin D can be selectively oxidized as follows to produce an aldehyde derivative:

$AMD-CH_3 + [O]$ -----> $AMD-CHO + H_2O$.

The invention also provides for sulfonic acid derivatives and a method of producing sulfonic acid derivatives. The two aromatic hydrogen atoms of actinomycin D can be reacted with compounds such as chlorosulfonic acid ($ClSO_3H$) to produce the sulfonic acid derivative. The reaction can be depicted as follows:

$AMD-H + ClSO_3H$ -----> $AMD-SO_3H + HCl$

where AMD-H represents either of the two aromatic hydrogen atoms of actinomycin D.

The invention provides for actinomycin D conjugates containing all of the above actinomycin D derivatives coupled to a target cell binding protein and for methods of producing such conjugates. All of the above actinomycin D derivatives can be coupled to a target cell binding protein by a variety of means. Such coupling reactions can be achieved at a position on actinomycin D, such as the derivatized moiety, and under conditions which do not affect the function of actinomycin D, and on the antibody at a site which maintains the specificity and affinity of the antigen binding site. The actinomycin D conjugates coupled in such a way should preferably be stable to physiological conditions present during administration to a patient and transport to the target cell location. Covalent attachment of the actinomycin D derivative to the target cell binding protein is preferable for such stability.

Coupling of isocyanate and thioisocyanate derivatives of actinomycin D to a target cell binding protein

can be accomplished, for example, via reaction of the isocyanate moiety with a hydroxyl or primary amine reactive group on the target cell binding protein. Of these two reactive groups, the primary amine is preferred for reaction with the isocyanate. The reaction between the isocyanate and the hydroxyl or primary amine groups tends to occur rapidly and is therefore beneficial for the efficient production of the actinomycin D conjugates.

In a similar manner, the carboxylic acid derivatives of actinomycin D can also be coupled directly to a target cell binding protein. For these actinomycin D derivatives, the carboxylic acid moiety can be, for example, activated prior to coupling with a primary amine on the protein to produce an amide bond between the actinomycin D derivative and the target cell binding protein. Coupling of the carboxylic acid derivative through other reactive groups can also be used to form an actinomycin D conjugate and are known to one skilled in the art.

In a similar manner, aldehyde and sulfonic acid derivatives of actinomycin D can be directly coupled to a target cell binding protein to produce actinomycin D conjugates. Both of these derivatives are reactive with primary amine groups of lysines, for example. Aldehydes can be coupled through a schiff base formation while sulfonic acid derivatives can be activated prior to coupling.

Formation of actinomycin D conjugates with methylene bromide derivatives of actinomycin can be accomplished using spacers. The spacer should be selected so that one end is reactive with the methylene moiety of the methylene bromide group and the other end able to be coupled to a target cell binding protein as defined above. A spacer such as 4-carboxy-sodium-phenolate (NaO-Ar-COOH), which contains an aromatic oxygen atom at one end for reaction with methylene bromide and a carboxylic acid at the other end, is one such example.

The end on the spacer containing the carboxylic acid moiety remains free after coupling to methylene bromide derivatives of actinomycin D and available for coupling to a target cell binding protein. Alternatively, the spacer can first be attached to a target cell binding protein and then coupled to a methylene bromide derivative of actinomycin D. Reactive groups other than carboxylic acid moieties can also be used on the free end of the spacer. For example, primary amines can be used to couple the free end of a spacer to a target cell binding protein and thereby form an actinomycin D conjugate.

The invention also provides for the use of spacers to produce actinomycin D conjugates with isocyanate, thioisocyanate, carboxylic acids, aldehydes and sulfonic acid derivatives of actinomycin D as well. The spacer molecule can be of a variety of types depending upon the actinomycin D derivative being coupled to the target cell binding protein. The preferred features being that the spacer contain reactive groups appropriate for the specific derivative being coupled. For example, to form a conjugate with an isocyanate or thioisocyanate derivative, the spacer should contain at one end a hydroxyl, or more preferably, a primary amine for attachment through the isocyanate or thioisocyanate moiety of the derivative. The other end can contain, for example, a carboxylic acid for coupling to a target cell binding protein.

Likewise, a spacer can be used with carboxylic acid derivatives of actinomycin D so long as one end can react with the carboxylic acid moiety, such as a primary amine and the other end is able to be coupled to a target cell binding protein. Other examples of heterobifunctional spacers for carboxylic acid derivatives of actinomycin D can include aminocarboxylic acids, mercaptocarboxylic acids, and hydroxycarboxylic acids. These spacers yield intermediates having either urea, thiourethane or urethane spacer groups, respectively. The terminal carboxylic acid moiety of such spacers can be activated to form amide bonds with lysine moieties, for example, of a target cell binding protein. Aldehydes and sulfonic acid derivatives can be similarly coupled through appropriate reactive groups on spacers. Analogous reactions to those described above for directly coupling aldehydes and sulfonic acid derivatives to a target cell binding protein can be used to couple, for example, to a spacer and then to a target cell binding protein.

The order of reaction used for coupling an actinomycin D derivative to a target cell binding protein is determined by the specific chemistries of bonding used and follow either the sequence of first attaching the linker to the actinomycin D derivative and finally attaching to the target cell binding protein, or first attaching the linker to the target cell binding protein followed by attachment to the actinomycin D derivative. The chemistry and order for the above coupling reactions is well known to one skilled in the art and is reviewed in M. J. Embleton and M.C. Garnett, Monoclonal Antibody for Cancer Detection and Therapy, Academic Press 16:317-343 (1985); Ghose et al., Methods of Enzymology, Academic Press 93:280-338 (1983); H. Tae, Bifunctional Reagents, Methods Enz. 91:580 (1983), all of which are incorporated herein by reference.

Since actinomycin D works by acting upon DNA molecules, it can be coupled to any target cell binding protein which is selective for a molecule on a DNA containing target cell. Such molecules can include, for example, viral or bacterial antigens. A derivative of actinomycin D can be prepared for coupling, for example, to any antibody or antibody fragment of the desired target specificity. Actinomycin D derivatives

can also be prepared for coupling to other types of target cell binding proteins, such as hormones, growth factors and cell surface binding proteins of various types. All of these types of proteins demonstrate binding specificity toward specific target cell molecules.

The actinomycin D conjugates of the present invention can be used to produce cytotoxic effects on target cells. For example, it is possible to use actinomycin D conjugates to destroy a certain population of cells such as T cells or B cells in an organism by coupling an actinomycin D derivative to a target cell binding protein which exhibits binding specificity to the desired population of T or B cells. In a similar manner, a specific cell type can be purified from a contaminating population of cells in culture by treating the culture with an actinomycin D conjugate in which the target cell binding protein has binding specificity for the contaminating cell type. Therefore, the invention provides a method of producing cytotoxic effects on target cells by administering to the target cells an effective amount of an actinomycin D conjugate which exhibits selective binding to target cells. The conjugate can be combined with a pharmaceutically acceptable carrier.

The following examples are intended to illustrate, but not limit, the invention.

## EXAMPLE I

### Reaction of Actinomycin D with Oxalyl Chloride

In a 10 ml round bottom flask 10 mg actinomycin D was dissolved in 1 ml butyl acetate and 1 ml chlorobenzene was then added. The solution, which turned red, was stirred while 20 µl oxalyl chloride was added. A gas evolved vigorously and the solution turned dark red. The flask was heated for 0.1 hours, then another 20 µl of oxalyl chloride was added. The color changed from dark blood red to pale yellow. The mixture was warmed and stirred for 18 hours. A 20 µl sample was removed and placed on a NaCl disc. Solvent was evaporated and infrared spectroscopy (IR) was conducted. Sharp peaks were observed at 2360 and 2341 corresponding to NCO groups of the newly formed isocyanate derivative of actinomycin D.

The residue after vacuum distillation was a yellow solid.

The above reaction was repeated using ethyl acetate as the initial solvent for the actinomycin D and heat. The product was evaporated and redissolved in dimethyl acetate (DMAc) (10 mg/300 µl).

## EXAMPLE II

### Reaction of Actinomycin D - Oxalyl Chloride Derivative with Antibody

Reaction of monoclonal antibody 9.2.27, 1 ml (6 mg) was done with 100 µl of DMAc solution containing the isocyanate derivative of actinomycin D (10 mg/500 µl, 2 mg actinomycin D), prepared as indicated in Example I. The conjugate mixture was gel filtered on a PD-10 column (Pharmacia, Piscataway, NJ), as recommended by the manufacturer.

After gel filtration, the second half of this sample, 0.5 ml, was reacted with 3 mg of carbodiimide and the pH was lowered to 6.7. The reaction was allowed to proceed for 2 hours and the material was again gel filtered on a PD-10 column.

The resulting conjugate was analyzed and the molar ratio of actinomycin D to the antibody was calculated to be 17, based on $\epsilon_{\text{actinomycin D}} = 6.5 \times 10^6$, $OD_{437\ nm}$ (optical density) actinomycin D = 0.371, 10:1 dilution; antibody concentration 5 mg/ml, MW (molecular weight) 155,000 g/mole.

## EXAMPLE III

### Methyl Group Bromination of Actinomycin D

Bromination with N-bromosuccinimide (NBS) is accomplished by adding 2.24 mmoles of actinomycin D in chlorobenzene to 2 mmoles of NBS and a trace of dibenzoyl peroxide. The mixture is heated to 80°C and allowed to react for 6 hours. The yield of AMD-$CH_2Br$ is 70-80%. The brominated derivative is then reacted further with aminocaprioic acid to yield an AMD-aminocaproic acid complex (AMD-$CH_2NH(CH_2)_5COOH$).

## EXAMPLE IV

### Oxidation of Methyl Groups on Actinomycin D

Equal molar amounts of actinomycin D and ceric ammonium nitrate in 50% aqueous acetic acid is heated at 60°C for 24 hours to produce the aldehyde derivative in 60% yield. The coupling of the aldehyde derivative to antibody is done through lysine groups by adding 20 µl 200 mM carbonate buffer to the aldehyde derivative at pH 9.5 and 8 mg of antibody. The mixture is stirred for 2 hours at room temperature. Then, 0.1 µl of sodium borohydride (4 µg/µl in water) and incubating 2 hours at 4°C.

EXAMPLE V

Sulfonic Acid Derivatives of Actinomycin D

A solution of actinomycin D in dimethylformamide (DMF) is cooled to 0°C and an equal molar amount of chlorosulfonic acid in methylene chloride is added dropwise. The reaction mixture is warmed to room temperature and purged with nitrogen until all Hcl is removed yielding the sulfonic acid derivative.

EXAMPLE VI

Immunoreactivity of Actinomycin D conjugates

To assess whether the actinomycin D conjugates retain binding activity and antigen specificity, an ELISA is performed on target and non-target cell lines. The target cell line expresses antigens which are specifically recognized by the target cell binding protein which is coupled to actinomycin D derivatives. The non-target cell lines do not express such antigens.

Appropriate cell lines that are in logarithmic growth phase are harvested using 1 mM EDTA. The cells are centrifuged at 1000 x g and rinsed 2 times with 1 x PBS. The cell pellet is resuspended in 1 x PBS in a volume to equal 1 x $10^6$ cells/ml. Using a 12 channel micropipettor, cells are platted out in 50 µl/well volume to equal 5 x $10^4$ cells/well in flexible 96 well plates. The 96 well plates are placed in a dry 37°C incubator for 3 to 5 days until the wells are completely dry. The plates are tested with purified monoclonal antibody standards before using in specificity assays.

The above prepared dry cell plates (both target and non-target cells) are coated with 200 µl of 0.5% BSA in PBS (0.5% BAS/PBS. The plates are placed on a shaker for 1 hour at room temperature and then washed with 0.5% BSA/PBS in the following manner. For the first wash, 300 µl of 0.5% BSA/PBS is added and immediately flicked out followed by slapping dry in a teri towel. For the remaining washes, 300 µl of 0.5% BSA/PBS is added and allowed to incubate at room temperature for 3-5 minutes before flicking dry. Following the final wash, the plates are slapped dry on a teri towel.

To the above prepared and washed plates, standards and samples are added in 100 µl volumes/well starting at 10 µg/ml with serial dilutions down to 1 pg/ml. The samples are diluted in 0.5% BSA/PBS and are incubated for 1 hour at room temperature while shaking. Following incubation, the plates are washed as described above and 100 µl/well of secondary antibody (goat anti-mouse coupled to horseradish peroxide (BioRad Laboratories, Richmond, CA) is added at 1:4000 dilution in 0.5% BSA/PBS. The antibody is allowed to bind for 30 minutes while shaking at room temperature. The plates are again washed as described above and peroxidase staining is developed for 5-20 minutes with shaking by addition of 100 µl/well of OPD/Phos-citrate buffer (40 ml of phosphate-citrate buffer, 12 mg o-phenylene-diamine, 6 µl $H_2O_2$). Reactions are quenched by addition of 50 µl/well of 4N sulfuric acid and read at 490 nm on a titertek Multiskan MCC/340 elisa reader.

EXAMPLE VII

In Vitro Cytotoxicity of Actinomycin D Conjugates

Thymidine incorporation into DNA was used as a measure of viable, dividing cells after treatment with an actinomycin D conjugate.

The conjugate prepared in Example II was evaluated for in vitro cytotoxicity as follows. $10^4$ M21-UCLA melanoma cells were grown in individual wells of a 96-well plate, at 37°C with 10% $CO_2$. The cells were grown in 100 µl of RPMI 1640 (GIBCO, Grand Island, NY) and 10% Fetal Bovine Serum (FBS). After 24 hours, the media was removed and replaced with 100 µl of RPMI 1640 containing concentrations ranging from $10^{-12}$ to $10^{-5}$ M of the immunoconjugate. After a 24 hour incubation 1 µCi/well of $^3$H-thymidine was added to measure DNA synthesis. The cells were labeled for 24 hours and the plates were then shock frozen at -70°C, thawed at 37°C and the cells were harvested using a PHD cell harvester (Cambridge

Technology, Inc., Cambridge, MA) as recommended by the manufacturer. The filters from the collected samples were placed in 4 mls of Ecolune scint (ICN Biomedicals, Costa Mesa, CA) and placed in a Beckman liquid scintillation counter (Beckman, Carlsbad, CA). The results of this in vitro assay indicated a high level of cytotoxicity for the antibody-actinomycin D conjugate.

EXAMPLE VIII

Iodination of the Conjugate

The immunoconjugates prepared as described in Example II were iodinated using IodoGen from Pierce (Pierce, Rockford, IL). 2 mg of IodoGen was dissolved in 4.7 ml of chloroform and between 120-240 µl of this solution was aliquoted into polystyrene tubes for iodination. The tubes were placed on ice and between 50-500 µg of actinomycin D conjugate in 100 µl was added. $Na^{125}I$ was added to 0.5-1.0 µCi and mixed. The mixture was incubated on ice for 25 minutes with gentle shaking. Unreacted $^{125}I$ was removed from the labeled actinomycin D conjugate by gel filtration in PBS using a PD10 column (Pharmacia, Pleasant Hill, CA).

EXAMPLE IX

In Vivo Binding of Conjugate

The antibody-actinomycin D conjugate from Example II was evaluated for in vivo binding specificity and affinity.

Thymus deficient BALBc (nude/nude) mice were subcutaneously injected with $2x10^6$ M21-UCLA melanoma cells. After two weeks, 3 µCi of $^{125}I$ immunoconjugate prepared as described in Example VIII, was injected into the tail vein. At 48 and 168 hours the animals were sacrificed and the radioactivity in individual organs was determined by Scintillation counting. The results are shown in Figure 2 and are represented as the percent of injected radioactivity per gram of tissue. The 48 hour samples were determined from the average of four animals. The 168 hour samples were determined from the average of three animals. The tissues indicated are: BL, blood; TA, tail; TU, tumor; HE, heart; SK, skin; MU, muscle; BO, bones; LU, lung; LI, liver; SP, spleen; ST, stomach; TH, thyroid; KI, kidney; IN, intestine.

The in vivo biodistribution data obtained with tumor bearing nude mice showed that the conjugate had a high degree of binding specificity and affinity. Further, the data indicated that any unbound conjugate was cleared from the body as shown by the relatively lower levels of conjugate found in the liver, kidney, spleen and intestine.

Although the invention has been described with reference to the presently preferred embodiment, it should be understood that various modifications can be made without departing from the spirit of the invention. Accordingly, the invention is limited only by the claims.

## Claims

**1.** An actinomycin D derivative of the formula

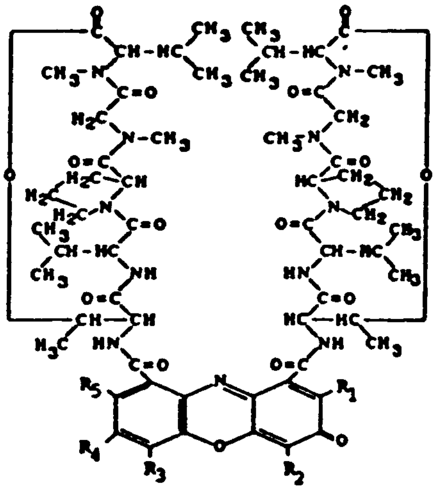

wherein $R_1$ is NCO, $NH_2$ $NHCH_2CO_2H$, or NHCORX where R is a hydrocarbon and X is selected from the group consisting of NCO, $CO_2H$, NCS;
$R_2$ and $R_3$ are $CH_3$, $CH_2Br$ or CHO;
$R_4$ and $R_5$ are H or $SO_3H$ with the proviso that the combination of groups at $R_1$, $R_2$, $R_3$, $R_4$ and $R_5$ does not yield actinomycin D as shown in Figure 1a.

**2.** The composition of claim 1, wherein said actinomycin D derivative is coupled to a target cell binding protein through the derivatized moiety.

**3.** The composition of claim 2, wherein said target cell binding protein is selected from the group consisting of an antibody, hormone, growth factor and cell surface binding protein, or functional fragment thereof.

**4.** The composition of claim 1, wherein said actinomycin D derivative is coupled to a spacer through the derivatized moiety.

**5.** The composition of claim 1, wherein said actinomycin D derivative is coupled through a spacer to a target cell binding protein, said coupling is through the derivatized moiety on actinomycin D.

**6.** The composition of claim 5, wherein said target cell binding protein is selected from the group consisting of an antibody, hormone, growth factor and cell surface binding protein, or functional fragment thereof.

**7.** A method of producing an isocyanate derivative of actinomycin D comprising combining actinomycin D with oxalyl chloride at a temperature sufficient to form said isocyanate derivative and to release carbon monoxide and hydrogen chloride.

**8.** A method of producing a carboxylic acid derivative of actinomycin D comprising treating actinomycin D with bromoacedic acid at a temperature sufficient to form said carboxylic acid derivative and to release hydrogen bromide.

**9.** A method of producing a methylene bromide derivative of actinomycin D comprising:
(a) combining actinomycin D with bromine or bromotrifluromethane to form a mixture; and
(b) treating the mixture with ultraviolet light.

**10.** A method of producing a methylene bromide derivative of actinomycin D comprising combining actinomycin D with N-bromosuccinimide at a temperature sufficient to form said methylene bromide derivative and to release succinimide.

**11.** A method of producing an actinomycin D derivative selected from the group consisting of isocyanate, carboxylic acid and thioisocyanate comprising combining actinomycin D with an acid chloride comprising the structure Cl-CO-R-X, wherein R is a hydrocarbon, X is selected from the group consisting of isocyanate, carboxylic acid and thioisocyanate, at a temperature sufficient to form said derivative and to release hydrogen chloride.

**12.** A method for producing an aldehyde derivative of actinomycin D comprising treating actinomycin D with an oxidizing agent at sufficient temperature to form said aldehyde derivative.

**13.** A method of producing a sulfonic acid derivative of actinomycin D comprising treating actinomycin D with chloro-sulfonic acid at sufficient temperature to form said derivative.

**14.** A method of producing an actinomycin D conjugate comprising coupling a target cell binding protein with a derivative of actinomycin D selected from the group consisting of isocyanate, carboxylic acid, thioisocyanate, methylene bromide, aldehyde or sulfonic acid.

**15.** The method of claim 14, wherein said target cell binding protein is selected from the group consisting of an antibody, hormone, growth factor and cell surface binding protein, or functional fragment thereof.

**16.** The method of claim 14, wherein said actinomycin D conjugate is formed directly through the derivatized moiety of actinomycin D and a reactive group on the target cell binding protein with the proviso that said derivatized moiety is not methylene bromide.

**17.** The method of claim 14, wherein said actinomycin D conjugate is formed through the derivatized moiety of actinomycin D and a reactive group on a spacer.

**18.** A method of producing an actinomycin D conjugate comprising:
(a) coupling a methylene bromide derivative of actinomycin D with a spacer to form an actinomycin D-spacer complex; and
(b) coupling said actinomycin D-spacer complex with a target cell binding protein.

**19.** The method of claim 18, wherein said target cell binding protein is selected from the group consisting of an antibody, hormone, growth factor and cell surface binding protein, or functional fragment thereof.

**20.** A method of producing cytotoxic effects on target cells comprising combining an effective amount of an actinomycin D conjugate in a pharmaceutically acceptable carrier, said conjugate having selective binding for said target cells; and administering said conjugate to target cells.

**21.** The method of claim 20, wherein said target cells are in a mammalian host.

**22.** The method of claim 20, wherein said actinomycin D conjugate is administered by a technique selected from the group consisting of intravenous, subcutaneous, and intraperitoneal.

**23.** A method of purifying cells comprising administering to a culture an effective amount of an actinomycin D conjugate having selective binding for contaminating target cells.

FIGURE 1

A.

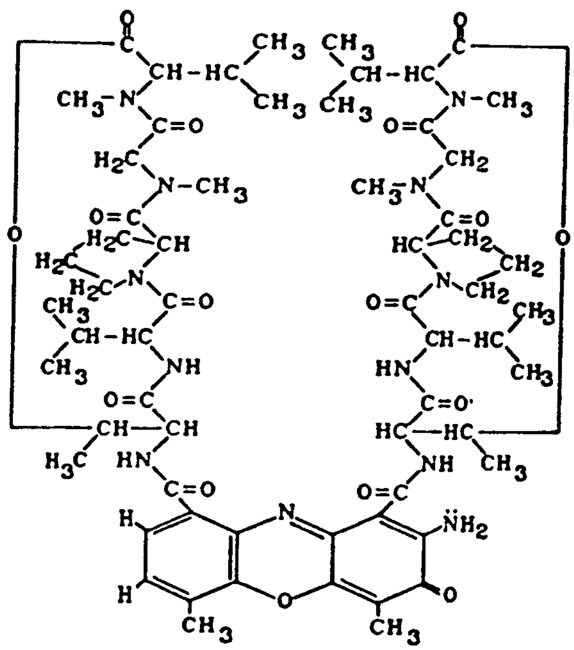

B.

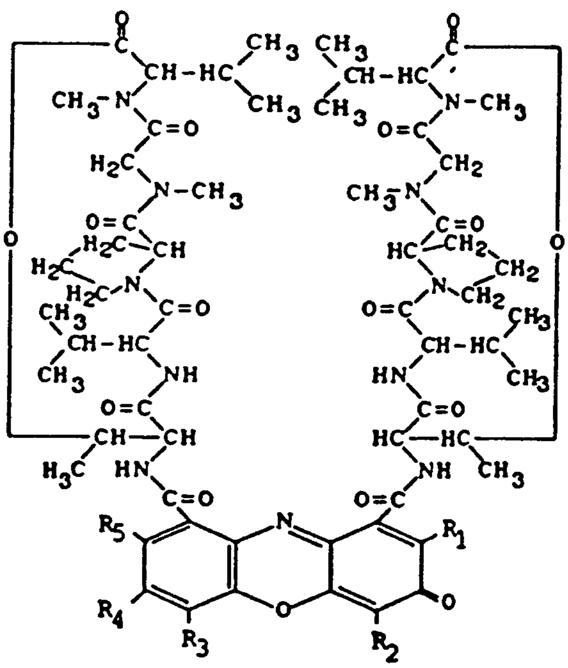

FIGURE 2

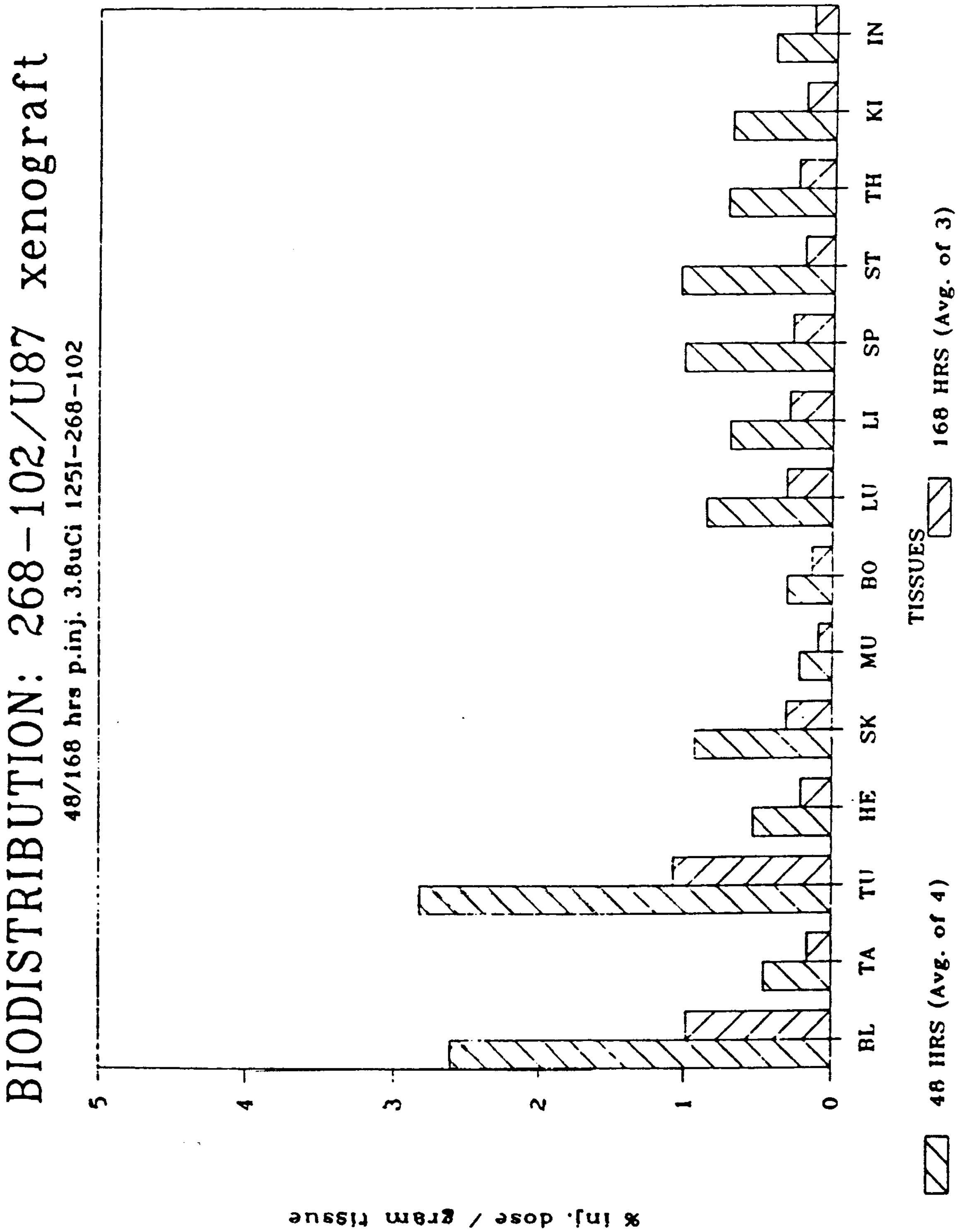
BIODISTRIBUTION: 268-102/U87 xenograft
48/168 hrs p.inj. 3.8uCi 125I-268-102
% inj. dose / gram tissue
0
1
2
3
4
5
BL
TA
TU
HE
SK
MU
BO
LU
LI
SP
ST
TH
KI
IN
TISSUES
48 HRS (Avg. of 4)
168 HRS (Avg. of 3)

European Patent Office

**PARTIAL EUROPEAN SEARCH REPORT**
which under Rule 45 of the European Patent Convention shall be considered, for the purposes of subsequent proceedings, as the European search report

Application number: EP 91114726.2

## DOCUMENTS CONSIDERED TO BE RELEVANT

| Category | Citation of document with indication, where appropriate, of relevant passages | Relevant to claim | CLASSIFICATION OF THE APPLICATION (Int. Cl.4) |
|---|---|---|---|
| X | PATENT ABSTRACTS OF JAPAN, unexamined applications, C field, vol. 9, no. 171, July 16, 1985 THE PATENT OFFICE JAPANESE GOVERNMENT page 39 C 291 * Kokai-no. 60-41 617 (TEIJIN K.K.) * -- | 1-3,20 | C 07 K 5/12<br>A 61 K 39/395<br>A 61 K 39/44<br>A 61 K 39/385 |
| X | EP - A - 0 224 885 (WAKUNAGA) * Abstract; description page 5; claims 1,2 * -- | 1-3,20 | |
| A | CHEMICAL ABSTRACTS, vol. 104, no. 14, April 7, 1986, Columbus, Ohio, USA Y. HASHIMOTO et al. "An approach to cancer chemotherapy by application of monoclonal antibody-modified liposomes." page 394, right column, | 1-3,20 | |

TECHNICAL FIELDS SEARCHED (Int. Cl.4)

C 07 K
A 61 K

## INCOMPLETE SEARCH

The Search Division considers that the present European patent application does not comply with the provisions of the European Patent Convention to such an extent that it is not possible to carry out a meaningful search into the state of the art on the basis of some of the claims.

Claims searched completely: 1-20,23
Claims searched incompletely: -
Claims not searched: 21,22
Reason for the limitation of the search:

Art. 52(4) EPC

| Place of search | Date of completion of the search | Examiner |
|---|---|---|
| VIENNA | 26-11-1991 | AUGUSTIN |

CATEGORY OF CITED DOCUMENTS

X : particularly relevant if taken alone
Y : particularly relevant if combined with another document of the same category
A : technological background
O : non-written disclosure
P : intermediate document
T : theory or principle underlying the invention
E : earlier patent document, but published on, or after the filing date
D : document cited in the application
L : document cited for other reasons
& : member of the same patent family, corresponding document

European Patent Office

**PARTIAL EUROPEAN SEARCH REPORT**

Application number

-2-

EP 91114726.2

| DOCUMENTS CONSIDERED TO BE RELEVANT | | | CLASSIFICATION OF THE APPLICATION (Int. Cl.4) |
|---|---|---|---|
| Category | Citation of document with indication, where appropriate, of relevant passages | Relevant to claim | |
| | abstract-no. 116 021c<br>& Int. Congr. Ser.-Excerpta Med. 1985, 690, 231-9<br>-- | | |
| A | CHEMICAL ABSTRACTS, vol. 100, no. 16, April 16, 1984, Columbus, Ohio, USA<br>Y. HASHIMOTO et al."Antitumor effect of actinonycin D entrapped in liposanes bearing subunits of tumor--specific monoclonal immuno-globulin M antibody."<br>page 355, left column, abstract-no. 126 820u<br>& Cancer Res. 1983, 43(11), 5328-34<br>---- | 1-3, 20 | TECHNICAL FIELDS SEARCHED (Int. Cl.4) |